# EUROPÄISCHE PATENTSCHRIFT

(11) **EP 1 213 300 B1**
(45) Veröffentlichungstag und Bekanntmachung des Hinweises auf die Patenterteilung: **26.12.2007**
(21) Anmeldenummer: 01250379.3
(22) Anmeldetag: 26.10.2001
(51) Int. Cl.: C07K 14/72, C12N 15/12, C12N 15/866, C12N 5/10, C07K 16/28, C12Q 1/68, A61K 48/00, G01N 33/53, G01N 33/574, G01N 33/74

(54) **Neue humane Androgenrezeptor-Varianten**
Novel human androgen receptor variants
Nouvelles variantes du récepteur d'androgène humain

(30) Priorität: 30.11.2000 DE 10061161
(43) Veröffentlichungstag der Anmeldung: 12.06.2002
(73) Patentinhaber: Bayer Schering Pharma Aktiengesellschaft, 13353 Berlin (DE)
(72) Erfinder: Ahrens-Fath, Isabelle, 13467 Berlin (DE); Haendler, Bernard, 13465 Berlin (DE)

(56) Entgegenhaltungen:
- WO-A-98/05797
- SCHNEIKERT JEAN ET AL: "Androgen receptor-Ets protein interaction is a novel mechanism for steroid hormone-mediated down-modulation of matrix metalloproteinase expression." JOURNAL OF BIOLOGICAL CHEMISTRY, Bd. 271, Nr. 39, 1996, Seiten 23907-23913, XP002191480 ISSN: 0021-9258
- DOESBURG PAUL ET AL: "Functional in vivo interactions between the amino-terminal, transactivation domain and the ligand binding domain of the androgen receptor." BIOCHEMISTRY, Bd. 36, Nr. 5, 1997, Seiten 1052-1064, XP002191481 ISSN: 0006-2960
- GAO TIANSHU ET AL: "Transcriptional activation and transient expression of the human androgen receptor." JOURNAL OF STEROID BIOCHEMISTRY AND MOLECULAR BIOLOGY, Bd. 59, Nr. 1, 1996, Seiten 9-20, XP002191482 ISSN: 0960-0760
- CATALANO MARIA G ET AL: "Altered expression of androgen-receptor isoforms in human colon-cancer tissues." INTERNATIONAL JOURNAL OF CANCER, Bd. 86, Nr. 3, 1. Mai 2000 (2000-05-01), Seiten 325-330, XP002191483 ISSN: 0020-7136
- LUBAHN D B ET AL: "CLONING OF HUMAN ANDROGEN RECEPTOR COMPLEMENTARY DNA AND LOCALIZATION TO THE X CHROMOSOME" SCIENCE (WASHINGTON D C), Bd. 240, Nr. 4850, 1988, Seiten 327-330, XP001061679 ISSN: 0036-8075
- TAPLIN MARY-ELLEN ET AL: "Selection for androgen receptor mutations in prostate cancers treated with androgen antagonist." CANCER RESEARCH, Bd. 59, Nr. 11, 1. Juni 1999 (1999-06-01), Seiten 2511-2515, XP002191550 ISSN: 0008-5472
- GOTTLIEB BRUCE ET AL: "Update of the androgen receptor gene mutations database" HUMAN MUTATION, WILEY-LISS, NEW YORK, NY, US, Bd. 14, Nr. 2, 1999, Seiten 103-114, XP002173777 ISSN: 1059-7794
- IKONEN T ET AL: "Heterodimerization is mainly responsible for the dominant negative activity of amino-terminally truncated rat androgen receptor forms" FEBS LETTERS, ELSEVIER SCIENCE PUBLISHERS, AMSTERDAM, NL, Bd. 430, Nr. 3, 3. Juli 1998 (1998-07-03), Seiten 393-396, XP004258099 ISSN: 0014-5793

### SEQUENZPROTOKOLL

<110> Schering AG
<120> Neue Androgenrezeptor-Varianten
<130> 52011
<140>
   <141>
<160> 6
<170> PatentIn Ver. 2.1
<210> 1
   <211> 1329
   <212> DNA
   <213> Homo sapiens
<400> 1
   gctgcgagca gagaggggtt cctcggaggt catctgttcc atcttcttgc ctatgcaaat 60
   gcctgcctga agctgctgga ggctggcttt gtaccggact ttgtacaggg aaccagggaa 120
   acgaatgcag agtgctcctg acattgcctg tcactttttc ccatgatact ctggcttcac 180
   agtttggaga ctgccaggga ccatgttttg cccattgact attactttcc accccagaag 240
   acctgcctga tctgtggaga tgaagcttct gggtgtcact atggagctct cacatgtgga 300
   agctgcaagg tcttcttcaa aagagccgct gaagggaaac agaagtacct gtgcgccagc 360
   agaaatgatt gcactattga taaattccga aggaaaaatt gtccatcttg tcgtcttcgg 420
   aaatgttatg aagcagggat gactctggga gcccggaagc tgaagaaact tggtaatctg 480
   aaactacagg aggaaggaga ggcttccagc accaccagcc ccactgagga gacaacccag 540
   aagctgacag tgtcacacat tgaaggctat gaatgtcagc ccatctttct gaatgtcctg 600
   gaagccattg agccaggtgt agtgtgtgct ggacacgaca acaaccagcc cgactccttt 660
   gcagccttgc tctctagcct caatgaactg ggagagagac agcttgtaca cgtggtcaag 720
   tgggccaagg ccttgcctgg cttccgcaac ttacacgtgg acgaccagat ggctgtcatt 780
   cagtactcct ggatggggct catggtgttt gccatgggct ggcgatcctt caccaatgtc 840
   aactccagga tgctctactt cgcccctgat ctggttttca atgagtaccg catgcacaag 900
   tcccggatgt acagccagtg tgtccgaatg aggcacctct ctcaagagtt tggatggctc 960
   caaatcaccc cccaggaatt cctgtgcatg aaagcactgc tactcttcag cattattcca 1020
   gtggatgggc tgaaaaatca aaaattcttt gatgaacttc gaatgaacta catcaaggaa 1080
   ctcgatcgta tcattgcatg caaaagaaaa aatcccacat cctgctcaag acgcttctac 1140
   cagctcacca agctcctgga ctccgtgcag cctattgcga gagagctgca tcagttcact 1200
   tttgacctgc taatcaagtc acacatggtg agcgtggact ttccggaaat gatggcagag 1260
   atcatctctg tgcaagtgcc caagatcctt tctgggaaag tcaagcccat ctatttccac 1320
   acccagtga                                                         1329
<210> 2
   <211> 388
   <212> PRT
   <213> Homo sapiens
<400> 2
   Met Ile Leu Trp Leu His Ser Leu Glu Thr Ala Arg Asp His Val Leu
    1               5                  10                  15
   Pro Ile Asp Tyr Tyr Phe Pro Pro Gln Lys Thr Cys Leu Ile Cys Gly
               20                  25                  30
   Asp Glu Ala Ser Gly Cys His Tyr Gly Ala Leu Thr Cys Gly Ser Cys
           35                  40                  45
   Lys Val Phe Phe Lys Arg Ala Ala Glu Gly Lys Gln Lys Tyr Leu Cys
       50                  55                  60
   Ala Ser Arg Asn Asp Cys Thr Ile Asp Lys Phe Arg Arg Lys Asn Cys
   65                  70                  75                  80
   Pro Ser Cys Arg Leu Arg Lys Cys Tyr Glu Ala Gly Met Thr Leu Gly
                   85                  90                  95
   Ala Arg Lys Leu Lys Lys Leu Gly Asn Leu Lys Leu Gln Glu Glu Gly
               100                 105                 110
   Glu Ala Ser Ser Thr Thr Ser Pro Thr Glu Glu Thr Thr Gln Lys Leu
           115                 120                 125
   Thr Val Ser His Ile Glu Gly Tyr Glu Cys Gln Pro Ile Phe Leu Asn
       130                 135                 140
   Val Leu Glu Ala Ile Glu Pro Gly Val Val Cys Ala Gly His Asp Asn
   145                 150                 155                 160
   Asn Gln Pro Asp Ser Phe Ala Ala Leu Leu Ser Ser Leu Asn Glu Leu
                   165                 170                 175
   Gly Glu Arg Gln Leu Val His Val Val Lys Trp Ala Lys Ala Leu Pro
               180                 185                 190
   Gly Phe Arg Asn Leu His Val Asp Asp Gln Met Ala Val Ile Gln Tyr
           195                 200                 205
   Ser Trp Met Gly Leu Met Val Phe Ala Met Gly Trp Arg Ser Phe Thr
       210                 215                 220
   Asn Val Asn Ser Arg Met Leu Tyr Phe Ala Pro Asp Leu Val Phe Asn
   225                 230                 235                 240
   Glu Tyr Arg Met His Lys Ser Arg Met Tyr Ser Gln Cys Val Arg Met
                   245                 250                 255
   Arg His Leu Ser Gln Glu Phe Gly Trp Leu Gln Ile Thr Pro Gln Glu
               260                 265                 270
   Phe Leu Cys Met Lys Ala Leu Leu Leu Phe Ser Ile Ile Pro Val Asp
           275                 280                 285
   Gly Leu Lys Asn Gln Lys Phe Phe Asp Glu Leu Arg Met Asn Tyr Ile
       290                 295                 300
   Lys Glu Leu Asp Arg Ile Ile Ala Cys Lys Arg Lys Asn Pro Thr Ser
   305                 310                 315                 320
   Cys Ser Arg Arg Phe Tyr Gln Leu Thr Lys Leu Leu Asp Ser Val Gln
                   325                 330                 335
   Pro Ile Ala Arg Glu Leu His Gln Phe Thr Phe Asp Leu Leu Ile Lys
               340                 345                 350
   Ser His Met Val Ser Val Asp Phe Pro Glu Met Met Ala Glu Ile Ile
           355                 360                 365
   Ser Val Gln Val Pro Lys Ile Leu Ser Gly Lys Val Lys Pro Ile Tyr
   370 375 380
   Phe His Thr Gln
   385
<210> 3
   <211> 1171
   <212> DNA
   <213> Homo sapiens
<400> 3
   gctgcgagca gagaggggtt cctcggaggt catctgttcc atcttcttgc ctatgcaaat 60
   gcctgcctga agctgctgga ggctggcttt gtaccggact ttgtacaggg aaccagggaa 120
   acgaatgcag agtgctcctg acattgcctg tcactttttc ccatgatact ctggcttcac 180
   agtttggaga ctgccaggga ccatgttttg cccattgact attactttcc accccagaag 240
   acctgcctga tctgtggaga tgaagcttct gggtgtcact atggagctct cacatgtgga 300
   agctgcaagg tcttcttcaa aagagccgct gaagggaaac agaagtacct gtgcgccagc 360
   agaaatgatt gcactattga taaattccga aggaaaaatt gtccatcttg tcgtcttcgg 420
   aaatgttatg aagcagggat gactctggga gcccggaagc tgaagaaact tggtaatctg 480
   aaactacagg aggaaggaga ggcttccagc accaccagcc ccactgagga gacaacccag 540
   aagctgacag tgtcacacat tgaaggctat gaatgtcagc ccatctttct gaatgtcctg 600
   gaagccattg agccaggtgt agtgtgtgct ggacacgaca acaaccagcc cgactccttt 660
   gcagccttgc tctctagcct caatgaactg ggagagagac agcttgtaca cgtggtcaag 720
   tgggccaagg ccttgcctgg cttccgcaac ttacacgtgg acgaccagat ggctgtcatt 780
   cagtactcct ggatggggct catggtgttt gccatgggct ggcgatcctt caccaatgtc 840
   aactccagga tgctctactt cgcccctgat ctggttttca atgagtaccg catgcacaag 900
   tcccggatgt acagccagtg tgtccgaatg aggcacctct ctcaagagtt tggatggctc 960
   caaatcaccc cccaggaatt cctgtgcatg aaagcactgc tactcttcag cattaattgc 1020
   gagagagctg catcagttca cttttgacct gctaatcaag tcacacatgg tgagcgtgga 1080
   ctttccggaa atgatggcag agatcatctc tgtgcaagtg cccaagatcc tttctgggaa 1140
   agtcaagccc atctatttcc acacccagtg a                                1171
<210> 4
   <211> 294
   <212> PRT
   <213> Homo sapiens
<400> 4
   Met Ile Leu Trp Leu His Ser Leu Glu Thr Ala Arg Asp His Val Leu
   1 5 10 15
   Pro Ile Asp Tyr Tyr Phe Pro Pro Gln Lys Thr Cys Leu Ile Cys Gly
   20 25 30
   Asp Glu Ala Ser Gly Cys His Tyr Gly Ala Leu Thr Cys Gly Ser Cys
   35 40 45
   Lys Val Phe Phe Lys Arg Ala Ala Glu Gly Lys Gln Lys Tyr Leu Cys
   50 55 60
   Ala Ser Arg Asn Asp Cys Thr Ile Asp Lys Phe Arg Arg Lys Asn Cys
   65 70 75 80
   Pro Ser Cys Arg Leu Arg Lys Cys Tyr Glu Ala Gly Met Thr Leu Gly
   85 90 95
   Ala Arg Lys Leu Lys Lys Leu Gly Asn Leu Lys Leu Gln Glu Glu Gly
   100 105 110
   Glu Ala Ser Ser Thr Thr Ser Pro Thr Glu Glu Thr Thr Gln Lys Leu
   115 120 125
   Thr Val Ser His Ile Glu Gly Tyr Glu Cys Gln Pro Ile Phe Leu Asn
   130 135 140
   Val Leu Glu Ala Ile Glu Pro Gly Val Val Cys Ala Gly His Asp Asn
   145 150 155 160
   Asn Gln Pro Asp Ser Phe Ala Ala Leu Leu Ser Ser Leu Asn Glu Leu
   165 170 175
   Gly Glu Arg Gln Leu Val His Val Val Lys Trp Ala Lys Ala Leu Pro
   180 185 190
   Gly Phe Arg Asn Leu His Val Asp Asp Gln Met Ala Val Ile Gln Tyr
   195 200 205
   Ser Trp Met Gly Leu Met Val Phe Ala Met Gly Trp Arg Ser Phe Thr
   210 215 220
   Asn Val Asn Ser Arg Met Leu Tyr Phe Ala Pro Asp Leu Val Phe Asn
   225 230 235 240
   Glu Tyr Arg Met His Lys Ser Arg Met Tyr Ser Gln Cys Val Arg Met
   245 250 255
   Arg His Leu Ser Gln Glu Phe Gly Trp Leu Gln Ile Thr Pro Gln Glu
   260 265 270
   Phe Leu Cys Met Lys Ala Leu Leu Leu Phe Ser Ile Asn Cys Glu Arg
   275 280 285
   Ala Ala Ser Val His Phe
   290
<210> 5
   <211> 10
   <212> PRT
   <213> Homo sapiens
<400> 5
   Asn Cys Glu Arg Ala Ala Ser Val His Phe
    1               5                   10
<210> 6
   <211> 7
   <212> PRT
   <213> Homo sapiens
<400> 6
   Met Ile Leu Trp Leu His Ser
    1               5

## Beschreibung

Die Erfindung betrifft zwei neue Varianten des Androgenrezeptors und ihre Verwendung.

Androgene sind die männlichen Geschlechtshormone und werden hauptsächlich im Hoden produziert (Roy, A. K. et al., Vitam. Horm. 1999, 55, 309-352). Sie steuern die männliche sexuelle Differenzierung und sind für die Spermatogenese essentiell. Weiterhin sind Androgene für die Ausprägung der sekundären Geschlechtsmerkmale und das sexuelle Verhaltensmuster verantwortlich. Androgene spielen auch eine essentielle Rolle in der Entstehung und Vermehrung von Prostata- und Hodenkrebs (Craft, N. und Sawyers, C. L., Cancer Metastasis Rev. 1998-99, 17, 421-427; Rajperts-De Meyts E. und Skakkebaek, N. E., Eur. Urol. 1993, 23, 54-59).
Androgene wirken durch Bindung an einen spezifischen Kernrezeptor, den Androgenrezeptor. Der bereits bekannte Androgenrezeptor ist ein Ligand-abhängiger Transkriptionsfaktor, der eine Ligandenbindungstelle, eine DNA-Bindungstelle und mehrere Transaktivierungsfunktionen besitzt (Lindzey, J. et al., Vitam. Horm. 1994, 49, 383-432). Die Haupttransaktivierungsfunktion befindet sich in der N-terminalen Hälfte, die vom Exon 1 des Androgenrezeptorgens kodiert wird. Wenn der Ligand, ein Androgen, bindet, ändert sich die Konformation des Rezeptors. Durch diese Konformationsänderung kann der Rezeptor ein Dimer bilden und an eine spezifische doppelsträngige DNA Sequenz binden, die Steroid-Response Element heißt. Durch Interaktionen mit Koaktivatoren und anderen Transkriptionsfaktoren wird die Transkription des Zielgens aktiviert (Lindzey, J. et al., Vitam. Horm. 1994, 49, 383-432).
Androgene spielen eine Rolle bei Hormon-abhängigen Tumoren. So wird z.B. Prostatakrebs mit Antiandrogenen, die mit der Bindung der natürlichen Androgene an den Androgenrezeptor kompetieren, behandelt. Hierbei zeigt sich häufig, daß die Antiandrogene nach einer gewissen Behandlungszeit nicht mehr wirksam sind (Crawford, E. D. et al., Urology 1999, 54, 1-7). Als Ursachen für diese Therapieresistenz wurden Mutationen des Androgenrezeptors, die eine Stimulierung durch Antiandrogene bzw. durch Estrogene oder Glucocorticoide erlauben (Brinkmann, A. O. und Trapman, J., Nature Med. 2000, 6, 628-629), postuliert. Diese Mutationen kommen allerdings relativ selten vor. Eine weitere mögliche Ursache ist eine Amplifizierung des Androgenrezeptorgens, wie in etwa 28% der androgenresistenten Patienten beschrieben (Koivisto, P. et al., Cancer Res. 1997, 57, 314-319). Damit können bei weitem nicht alle Fälle erklärt werden. Für eine erfolgreiche Tumor-Therapie ist es deshalb wünschenswert, einen weiteren Angriffspunkt für die Therapie zu kennen.

Dieses Problem wurde gelöst durch die Bereitstellung von zwei neuen Varianten des Androgenrezeptors.

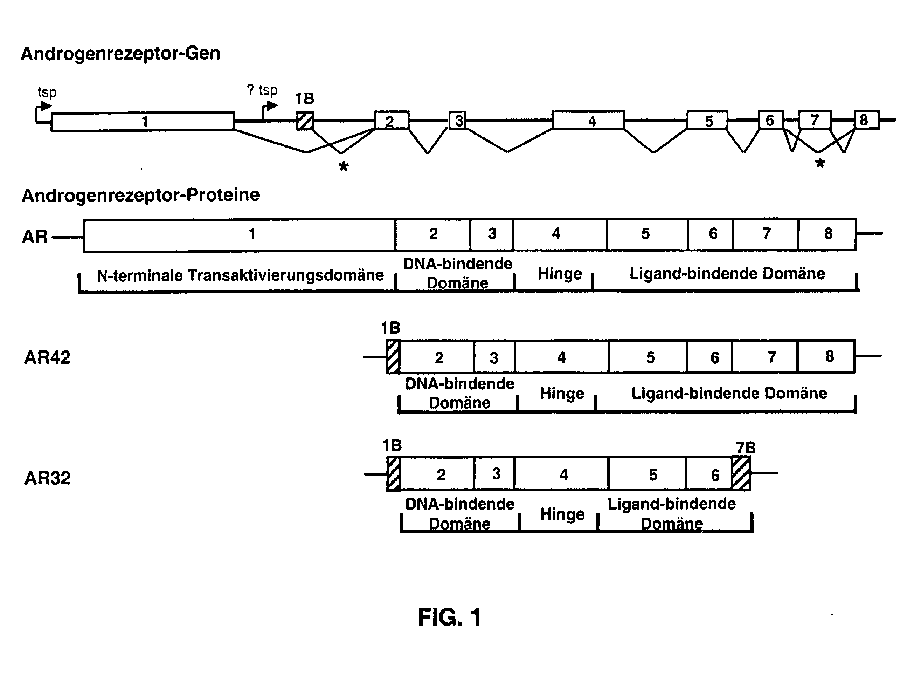

Der erste erfindungsgemäße Androgenrezeptor mit der in Seq ID NO 2 angegebenen Aminosäuresequenz wird im folgenden AR42 genannt und der erfindungsgemäße Androgenrezeptor mit der in Seq ID NO 4 angegebenen Aminosäuresequenz wird AR32 genannt. Der bereits bekannte Androgenrezeptor (Lubahn, D. B. et al., Science 1988, 240, 327-330; Chang et al., Science 1988, 240, 324-326) hat im folgenden die Bezeichnung AR.
Die Sequenzen von AR und AR42 stimmen im Bereich der DNA-bindenden Domäne, der sogenannten "hinge"-Domäne und der Liganden-bindenden Domäne überein (s. Exons 2-8 in FIG.1). Sie unterscheiden sich im Bereich des N-Terminus. Während der AR hier eine etwa 537 Aminosäure-lange Transaktivierungsdomäne hat, besitzt der AR42 hier einen nur 7 Aminosäure-langen Bereich, dessen Sequenz unterschiedlich zu der Sequenz der Transaktivierungsdomäne des AR ist. Dieser 7 Aminosäure-lange Bereich ist in der genomischen Sequenz in keinem bisher bekannten Exon enthalten; vielmehr ist er Bestandteil eines DNA-Bereichs, der bisher als nicht translatiert galt.

Der AR32 unterscheidet sich vom AR im N-Terminus und im C-Terminus (s. Exons 1, 7 und 8 in FIG.1). Der AR32 hat die gleiche N-terminale Sequenz wie AR42. Er unterscheidet sich vom AR42 und vom AR im C-Terminus. Seine C-terminale Sequenz ist kürzer und 10 Aminosäuren sind unterschiedlich verglichen mit AR42 und AR.

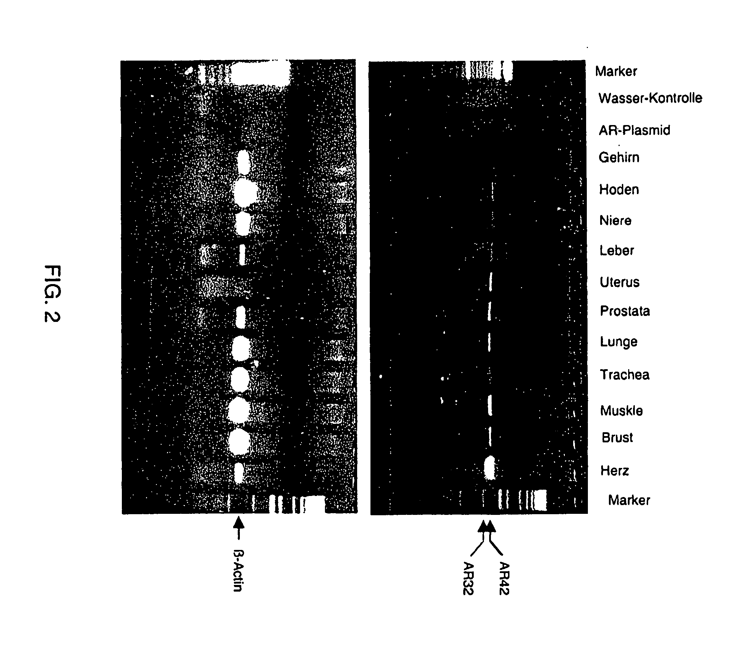

Die erfindungsgemäßen AR42 und AR32 sind in verschiedenen Geweben von gesunden Menschen exprimiert. AR42 ist besonders stark im Herzen exprimiert (FIG. 2).

Die erfindungsgemäßen AR42 und AR32 können Androgen und andere Liganden binden. Nach Bindung eines Liganden können AR42 und AR32 Homodimere (AR42/AR42 oder AR32/AR32) oder untereinander oder mit dem AR Heterodimere (AR42/AR oder AR32/AR) bilden. Die Homodimere können zwar an das Steroid- Response Element des AR binden; sie können jedoch nicht die Transkription von den Zielgenen aktivieren, die der AR aktiviert. AR42 und AR32 wirken dann als Repressor für den AR. Durch eine Heterodimer-Bildung mit dem AR kann die Aktivität des AR moduliert werden. Ob es sich um eine Inhibierung oder Aktivierung handelt, hängt von den Zielgenen ab. Eine Aktivierung der Expression erfolgt bei den Zielgenen, deren Expression durch eine Interaktion des AR mit Ets-Transkriptionsfaktoren blockiert ist. Diese Ets-Transkriptionsfaktoren fungieren als Corepressoren für den AR durch Bindung an dessen N-Terminus (Schneikert J. et al., J. Biol. Chem. 1996, 271, 23907-23913). Dagegen können sie nicht an den AR42 oder AR32 binden. Dadurch wird die Blockierung aufgehoben und die Expression stimuliert.

Die Erfindung betrifft Nukleinsäuren, die für einen Androgenrezeptor kodieren, wobei sie die in Seq ID NO 1 und/oder 3 dargestellten Nukleotidsequenzen umfassen.

Nukleinsäuren können einzel- oder doppelsträngige DNA, z.B. cDNA, oder RNA, z.B. mRNA, cRNA, pre-mRNA darstellen.

Ein Protein kodierender Abschnitt der in Seq ID NO 1 dargestellten Sequenz liegt im Bereich von Nukleotid 163 bis 1329 und ein Protein kodierender Abschnitt der in Seq ID NO 3 dargestellten Sequenz liegt im Bereich von Nukleotid NO 163 bis NO 1047.

Gegenstand der Erfindung sind ferner Nukleinsäuren, die für ein Polypeptid mit der in Seq ID NO 2 oder/und 4 dargestellten Aminosäuresequenz kodieren.

Die erfindungsgemäßen Nukleinsäuren sind aus Säugern, z.B. humanen Zellen oder aus einer cDNA-Bibliothek oder einer genomischen Bibliothek, die z.B. aus menschlichen Zellen gewonnen wird, erhältlich. Sie können nach bekannten Techniken unter Verwendung kurzer Abschnitte der in Seq ID NO 1 und 3 gezeigten Nukleinsäureseqenzen als Hybridisierungssonden oder Amplifikationsprimer isoliert werden. Besonders bevorzugt sind solche Abschnitte, die für die in Seq ID NO 5 oder 6 dargestellten Peptidsequenzen kodieren.

Die Erfindung betrifft weiterhin Polypeptide, die von einer erfindungsgemäßen Nukleinsäure kodiert werden. Diese Polypeptide haben die Funktion eines Androgenrezeptors.
Weiterhin sind Gegenstand der Erfindung Polypeptide, die die in Seq ID NO 2 oder 4 dargestellte Aminosäuresequenz umfassen.
Die erfindungsgemäßen Polypeptide können rekombinante Polypeptide, natürliche, isolierte Polypeptide oder synthetische Polypeptide sein.

Ferner betrifft die Erfindung Peptide, die die in Seq ID NO 5 dargestellte Sequenz umfaßen. Die in Seq ID NO 5 dargestellte Sequenz entspricht dem C-Terminus (Aminosäure 285-294) von AR32.

Die Erfindung betrifft auch Peptide, die die in Seq ID NO 6 dargestellte Aminosäuresequenz umfassen. Die in Seq ID NO 6 dargestellte Aminosäuresequenz entspricht dem N-Terminus (Aminosäure 1-7) von AR42 und AR32.

Die erfindungsgemäßen Polypeptide und die erfindungsgemäßen Peptide können zur Herstellung von Antikörpern verwendet werden. Zur Herstellung polyklonaler Antikörper können die Peptide z.B. an KLH (Keyhole Limpet Hemocyanin) gebunden werden und Tieren, z.B. Kaninchen, gespritzt werden. Sie können auch zur Herstellung monoklonaler Antikörper verwendet werden. Zur Antikörperherstellung kann ein erfindungsgemäßes Peptid oder eine Mischung mehrer erfindungsgemäßer Peptide verwendet werden. Die Herstellung der Antikörpern erfolgt dabei nach Standardverfahren wie sie z.B. in Kohler, G. und Milstein, C., Nature 1975, 256, 495-497 und Nelson, P. N. et al., Mol. Pathol. 2000, 53, 111-117 beschrieben sind.

Gegenstand der Erfindung sind auch die Antikörper, die gegen ein Polypeptid mit der Seq ID NO 5 oder ID NO 6 gerichtet sind.

Die erfindungsgemäßen Antikörper können zur Detektion der erfindungsgemäßen AR42 und AR32 verwendet werden. Dies kann z.B. durch Immunhistochemie erfolgen. Bevorzugt ist der Nachweis der erfindungsgemäßen Polypeptide in Tumorgewebe besonders in Gewebe von Prostatatumoren. So kann festgestellt werden, ob eine Hormontherapieresistenz auf eine veränderte Expression der erfindungsgemäßen AR42 und/oder AR32 zurückzuführen ist. Die erfindungsgemäßen Antikörper können auch in anderen Immuntests wie z.B. einem ELISA (enzyme linked immunosorbent assay) oder in Radioimmunotests eingesetzt werden. So kann die Konzentration an erfindungsgemäßen AR42 und AR32 in Gewebe-oder Zellextrakten nachgewiesen werden.

Der Nachweis der Expression von AR42 oder AR32 kann auch über den Nachweis von mRNA in den Zellen erfolgen. Gegenstand der Erfindung ist daher auch die Verwendung einer Sonde mit Nukleinsäuresequenzen, die komplementär zu den Nukleinsäuresequenzen sind, die für die erfindungsgemäßen Peptide mit einer Aminosäuresequenz gemäß Seq ID NO 5 oder 6 kodieren, zur Herstellung eines Reagenz zum Nachweis der Gegenwart von erfindungsgemäßer mRNA in Tumorzellen. Eine Sonde ist ein kurzes DNA-Stück mit mindestens 14 Nukleotiden. Die erfindungsgemäßen Sonden können z.B. in einer Northern Blot Analyse verwendet werden. Diese Methode ist z.B. in Sambrook, J. et al., 1989, Cold Spring Harbor Laboratory Press, beschrieben. Andere Methoden zum Nachweis der RNA sind In Situ Hybridisierung, RNAse Protection Assay oder PCR.

Weiterhin sind Gegenstand der Erfindung Vektoren die mindestens eine Kopie einer erfindungsgemäßen Nukleinsäure enthält. Vektoren können prokaryontische oder eukaryontische Vektoren sein. Beispiele für Vektoren sind pPRO (Clontech), pBAD (Invitrogen), pSG5 (Stratagene), pCl (Promega), pIRES (Clontech), pBAC (Clontech), pMET (Invitrogen), pBlueBac (Invitrogen). In diese Vektoren kann mit den dem Fachmann bekannten Methoden die erfindungsgemäßen Nukleinsäuren eingefügt werden. Die erfindungsgemäßen Nukleinsäuren befinden sich vorzugsweise in Verbindung mit Expressionssignalen wie z.B. Promotor und Enhancer auf dem Vektor.

Die Erfindung betrifft ferner Zellen, die mit einer erfindungsgemäßen Nukleinsäuresequenz oder mit einem erfindungsgemäßen Vektor transfiziert sind. Als Zellen können z.B. *E. coli,* Hefe, *Pichia,* Sf9, COS, CV-1 oder BHK verwendet werden. Bevorzugt sind Zellen, die aus der Gruppe bestehend aus PC-3-Zellen, LNCaP-Zellen, CV-1-Zellen und Dunning-Zellen ausgewählt werden. Diese Zellen können sowohl für die Produktion von AR42 und /oder AR32 als auch für Zell-basierte Tests verwendet werden.

Gegenstand der Erfindung ist weiterhin die Verwendung
a. einer erfindunggemäßen Nukleinsäure,
b. eines erfindungsgemäßen Polypeptids,
c. eines Peptids mit der in Seq ID NO 5 dargestellten Aminosäuresequenz oder
d. einer erfindungsgemäßen Zelle
zur Identifizierung von Effektoren eines erfindungsgemäßen Polypeptids. Effektoren sind Substanzen, die auf das erfindungsgemäße Polypeptid inhibitorisch oder aktivierend wirken, und die in der Lage sind, die Androgenrezeptor-Funktion der erfindungsgemäßen Polypeptide zu beeinflussen.

Weiterhin betrifft die Erfindung ein Testsystem zur Detektion von Inhibitoren oder Aktivatoren der Androgenrezeptorfunktion der erfindungsgemäßen Polypeptide, wobei
a. in den erfindungsgemäßen Zellen ein Reportergen exprimiert wird und
b. diese Zellen, wenn sie keinen oder nur wenig eines erfindungsgemäßen Polypeptids enthalten, zusätzlich mit einem erfindungsgemäßen Vektor transfiziert werden,
c. die Zellen in Gegenwart oder Abwesenheit der Testsubstanzen kultiviert werden und
d. die Veränderung der Expression des Reportergens gemessen wird.

Ferner betrifft die Erfindung ein Testsystem zur Detektion von Testsubstanzen mit antiandrogener Aktivität, wobei
a. in den erfindungsgemäßen Zellen ein Reportergen exprimiert wird und
b. diese Zellen, wenn sie keinen oder nur wenig eines erfindungsgemäßen Polypeptids enthalten, zusätzlich mit einem erfindungsgemäßen Vektor transfiziert werden,
c. die Zellen in Gegenwart oder Abwesenheit der Testsubstanzen bei gleichzeitiger Anwesenheit eines Androgens kultiviert werden und
d. die Veränderung der Expression des Reportergens gemessen wird

Für ein erfindungsgemäßes Testsystem werden geeignete Zellen, zum Beispiel CV-1-Zellen, COS-Zellen oder Zellen, die aus der Prostata stammen, stabil oder transient mit einer erfindungsgemäßen Nukleinsäure oder mit Teilen davon oder mit Teilen davon in Kombination mit einer Transaktivierungsdomäne von anderen Faktoren transfiziert. Teile einer erfindungsgemäßen Nukleinsäure können z.B. die Liganden-bindende Domäne, die Transaktivierungsdomäne und die DNA-bindende Domäne sein. Transaktivierungsdomänen von anderen Faktoren können z.B. die Liganden-bindende Domäne, die Transaktivierungsdomäne und die DNA-bindende Domäne des AR oder des Progesteronrezeptors, die Gal 4-Transaktivierungsdomäne oder die VP16-Transaktivierungsdomäne sein. Reporter-Plasmide können cotransfiziert werden. Diese beinhalten ein oder mehrere Steroid-Response Elemente, die Inverted Repeats der TGTTCT-Sequenz mit einem Spacer von drei Basenpaaren darstellen. Weiterhin können solche Response Elemente Direct Repeats der TGTTCT-Sequenz mit einem Spacer von drei bis fünf Basenpaaren sein. Abweichungen in der TGTTCT-Sequenz, wie in natürlichen Response Elements beschrieben, sind möglich (Kokontis, J. M. und Liao, S., Vitam. Horm. 1999, 55, 219-307). Stromabwärts liegt, in operativer Verknüpfung, ein minimal Promoter (Schenborn, E. und Groskreutz, D., Mol. Biotechnol. 1999, 13, 29-44) und ein heterologes Reportergen. Reporter-Plasmide können auch ein Promotor- oder Promotor-Teile aus bekannten androgenregulierten Gene beinhalten. Bevorzugt werden Gene die in der Prostata androgenabhängig exprimiert werden. Beispiele dafür sind das PSA-, Probasin- und C3(1)-Gen. Reportergene können z.B. sein Luciferase-Gen, Chloramphenicolacetyltransferasegen, Urokinasegen, Green Fluorescence Protein-Gen und β-Galaktosidasegen. Die Testsubstanzen werden bevorzugt aus der Gruppe der Androgen-Derivate ausgewählt. Diese Testsyteme können aber auch zum Screenen von großen Substanzbibliotheken eingesetzt werden. Für das Testsystem zur Detektion von Testsubstanzen mit antiandrogener Aktivität können in Schritt c. als Androgen z.B. R1881, Testosteron, Dihydrotestosteron und Testosteron-Derivate verwendet werden.
Bevorzugt werden solche Substanzen, die in einem erfindungsgemäßen Testsystem die Expression eines Reportergens verändern, aber nicht Effektoren des AR sind. Zur Bestimmung, ob die Substanzen Effektoren des AR sind, kann ein Testsystem verwendet werden, das analog zu dem erfindungsgemäßen Testsystem aufgebaut ist, wobei die Zellen statt mit einem erfindungsgemäßen Vektor mit einem Vektor, der die Nukleinsäure des AR enthält, transfiziert werden.
Effektoren, die die erfindungsgemäßen Polypeptide aber nicht den AR aktivieren, führen durch Heterodimerbildung der erfindungsgemäßen Polypeptide mit dem AR zu einer Inhibition des AR. Diese inhibitorische Wirkung läßt sich mit einem in Beispiel 5 beschriebenen Testsystem bestimmen. Eine Inhibition des AR ist wünschenswert bei allen Androgen-abhängigen Erkrankungen, z.B. zur Behandlung von Prostata-Tumoren und auch bei der männlichen Fertilitätskontrolle. Bei der männliche Fertilitätskontrolle kann durch Inhibition des AR z.B. die Expression von Genen, die für die Bildung reifer Spermien notwendig sind, inhibiert werden.

Weiterhin können Gene identifiziert werden, die selektiv durch Homodimere oder Heterodimere der erfindungsgemäßen Polypeptide reguliert werden. Diese Gene können durch gezielte Knock-Out und Knock-In Experimente identifiziert werden.

Bevorzugt sind Substanzen, die in den erfindungsgemäßen Testsystemen die ReportergenAktivität mindestens 10-fach steigern oder inhibieren. Eine Substanz, die durch ein erfindungsgemäßes Verfahren identifiziert wird, kann gegebenenfalls bezüglich metabolischer Stabilität, Aktivität in einem erfindungsgemäßen Testsystem und/oder Bioverfügbarkeit optimiert werden. Dafür können in der Chemie gängige Methoden verwendet werden.

### Beschreibung der Abbildungen

FIG. 1 zeigt die Intron-Exon-Struktur des AR-Gens und die Domänenstruktur des AR, AR42 und des AR32. Im Gen wurden der bekannte Transkriptionsstart (tsp) im Promoter und der putative 2. Transkriptionsstart (?tsp) vor dem Exon 1B mit Pfeilen gezeigt. Das neue Exon 1B ist straffiert. Sterne markieren Splicingereignisse die spezifisch zu der Generierung der mRNA für AR42 und AR32 führen. Im Protein wurden die verschiedenen Domänen gezeigt. Die neuen Regionen, die in AR42 und AR32 durch das alternative Splicing des AR-Gens dazukommen, sind straffiert.

FIG. 2 zeigt die Gewebe-Verteilung von AR42 und AR32 mRNA. Ein Sense Primer, der gegen das spezifische AR42 und AR32 Exon und ein Antisense Primer, der gegen die gemeinsame C-terminale Region gerichtet waren, wurden für die PCR-Amplifizierung verwendet. Gesamt-RNA wurde aus verschiedenen humanen Geweben gewonnen und mit einer Reverse Transkriptase umgeschrieben. Diese First-Strand cDNA diente als Template. Die PCR-Produkte wurden auf einem Agarosegel getrennt und mit Ethidiumbromid gefärbt. Die AR42 cDNA ist als starke Bande und die AR32 cDNA als sehr schwache Bande auf dem Gel zu erkennen.

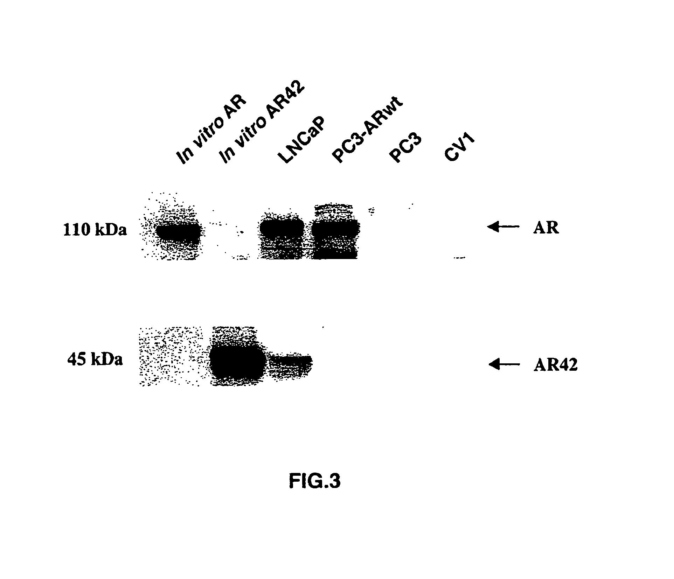

FIG. 3 zeigt die Expression von AR42 Protein in LNCaP Zellen. Ein Antikörper, der gegen die Ligand-bindende Domäne des AR gerichtet war, wurde für die Western Blot Analyse verwendet. Gesamt Zellextrakte aus verschiedenen Zelllinien (LNCaP, PC-3ARwt, PC-3, CV-1) wurden auf einem 8 % Tris-Glycine Gel aufgetragen. *In vitro* translatierte AR und AR42 Proteine wurden als Kontrolle aufgetragen. Das AR42 Protein wurde nur in LNCaP Zellen detektiert. Das 110 kDa AR Protein wurde in LNCaP und in PC3-ARwt detektiert. Die PC-3ARwt Zellinie wurde durch Transfizierung von PC-3 Zellen mit einem AR enthaltenden Plasmid erhalten.

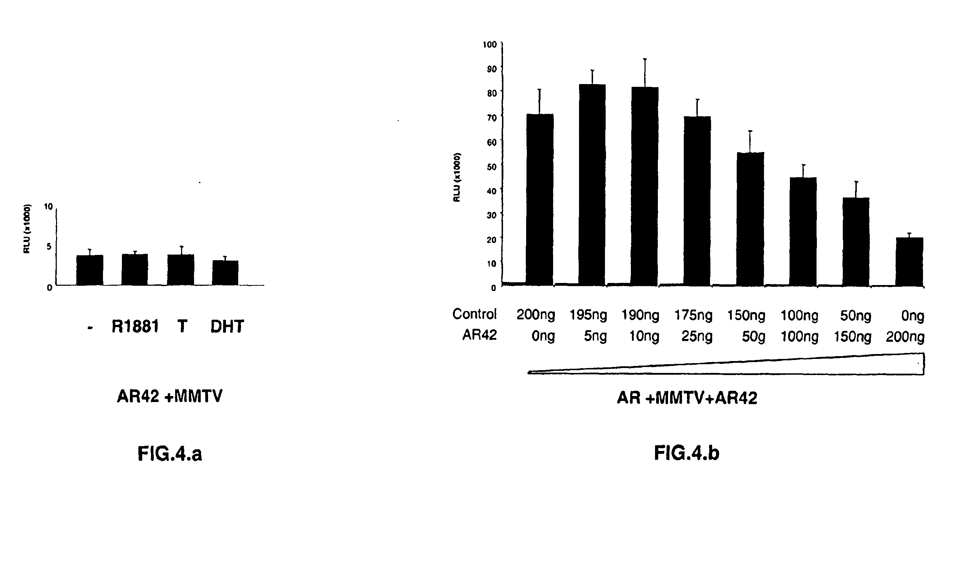

FIG. 4.a zeigt dass AR42 keine Transaktivierungsfunktion in PC-3 Zellen besitzt.
100 ng pSG5-AR42 wurden zusammen mit 100 ng eines Reporterplasmids, das den MMTV Promoter enthält, in PC-3 cotransfiziert. Diese Zellen wurden danach mit verschiedenen Androgenen (R1881: Metribolone; T: Testosteron; DHT: 5α-Dihydrotestosterone) mit einer Endkonzentration von 10⁻⁷ M behandelt.

FIG. 4.b zeigt die transreprimierende Aktivität von AR42. 10ng pSG5-AR und unterschiedliche Mengen an pSG5-AR42 wurden in PC-3 Zellen cotransfiziert. Zusätzlich wurden 100 ng eines Reporterplamids, das den MMTV promoter enthält, transfiziert. Die Reportergenaktivität wurde nach Behandlung mit R1881 (10⁻⁹ M) gemessen. Steigende Mengen an transfiziertem pSG5-AR42 hemmten die transaktivierenden Effekte von stimuliertem AR.

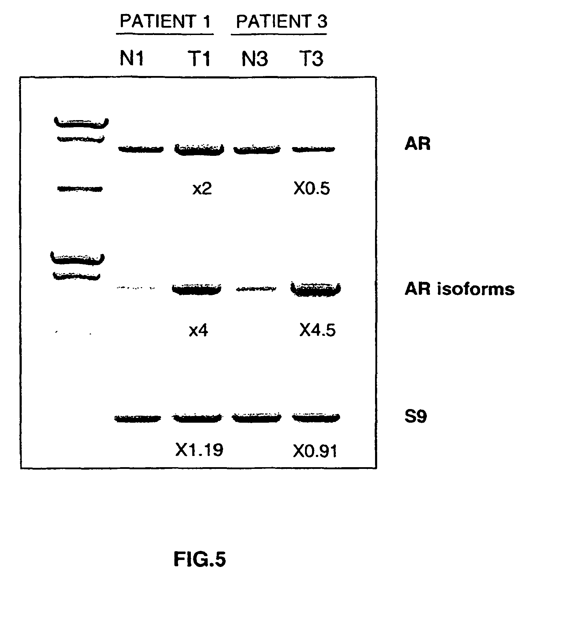

FIG. 5 zeigt die Expression von AR42 mRNA in Prostata-Tumor Gewebe. Gesamte Prostata-RNA wurde aus normalem (N) oder Tumor- (T) Gewebe von zwei Prostatacarcinoma-Patienten gewonnen und mit einer Reverse Transkriptase umgeschrieben. Ein Sense Primer, der gegen das spezifische AR42 und AR32 Exon und ein Antisense Primer, der gegen die gemeinsame AR C-terminale Region gerichtet waren, wurden für PCR-Amplifizierung verwendet. AR und S9 DNA-Fragmente wurden parallel reamplifiziert. Die S9 DNA-Mengen dienen als interner Standard. Die Ergebnisse zeigen, dass AR42-RNA in beiden Prostata-Tumor Geweben stärker exprimiert ist als in normalem Gewebe. Die AR-Transkriptmengen wiesen keine vergleichbare Änderungen auf. Die Bezeichnungen x2, x0.5 etc. zeigen an, um welchen Faktor die jeweilige Bande bei dem Tumorgewebe gegenüber dem Normalgewebe stärker ist.

### Beispiele

Die in den Beispielen verwendeten molekularbiologischen Methoden wie z.B. Polymerase-Kettenreaktion (PCR), Herstellung von cDNA, Klonierung von DNA, Sequenzierung von DNA, wurden wie in bekannten Lehrbüchern wie zum Beispiel in Molecular Cloning, A Laboratory Manual (Sambrook, J. et al., 1989, Cold Spring Harbor Laboratory Press) beschrieben, durchgeführt.

### Beispiel 1: Identifizierung und Klonierung von AR42 und AR32

Ausgangsmaterial war 1 µg gesamt-RNA aus humaner Placenta, die mittels des SMART RACE Amplification kits (Clontech) in cDNA umgewandelt wurde. Für die PCR-Amplifikation wurde der Advantage-2 PCR kit (Clontech) benutzt zusammen mit einem Antisense Primer (5'-CAGATTACCAAGCTTCAGCTTCCG-3'), der gegen die Hinge-Region des humanen Androgenrezeptors gerichtet ist, und einem Sense 5'-Smart II Primers verwendet. Die Reaktionsbedingungen waren: 5 Sek. 94 °C, 3 Min. 72 °C (5 Zyklen); 5 Sek. 94 °C, 10 Sek. 70 °C, 3 Min. 72 °C (5 Zyklen); 5 Sek. 94 °C, 10 Sek. 68 °C, 3 Min. 72 °C (27 Zyklen). Hierbei wurde ein Fragment von ca. 500 Basenpaaren amplifiziert, auf Agarosegel gereinigt, in das PCR-TOPO-Plasmid (Invitrogen) kloniert und sequenziert. Die DNA-Sequenz zeigte, daß die komplette DNA-bindende Domäne des Androgenrezeptors vorhanden war (entspricht Exon 2 und 3 im Androgenrezeptorgen). Zusätzlich war unmittelbar vor dem Exon 2 eine neue Sequenz angeknüpft. Dieser Abschnitt, den man als Exon 1B bezeichnen kann, beinhaltet ca. 160 Basenpaare aus dem untranslatiertem Bereich und eine kurze, für 7 Aminosäuren kodierende neue Sequenz. Um die komplette cDNA zu isolieren, wurden die Sense Primer 5'-ACAGGGAACCAGGGAAACGAATGCAGAGTGCTCCTGACATTGCCTGT-3'
(Endkonzentration 0,2 µM) und 5'-GACAGGGAACCAGGGAAACGAATG-3' (Endkonzentration 1 µM), die aus dem neuen Exon 1 B-Bereich stammen, und ein Antisense Primer (5'-TCACTGGGTGTGGAAATAGATGGGCTTGA-3'), der für das C-terminale Ende des bekannten AR kodiert, synthetisiert. Die vorgegebenen Bedingungen für die SMART-PCR wurden verwendet. Damit ist es möglich, ein Fragment von ca. 1200 Basenpaaren aus der gleichen Placenta cDNA zu amplifizieren und zu klonieren. Nach DNA Sequenzierung stellte sich heraus, daß es sich um zwei verschiedene Fragmente handelte, wie in Seq ID NO 1 und NO 3 gezeigt. In beiden war der neue, dem Exon 1B-entsprechenden Teil vorhanden. Der Unterschied zwischen AR42 und AR32 lag im C-terminale Bereich, da in AR32 die Region, die durch Exon 7 kodiert wird, fehlte. Eine Suche in genomischen Datenbanken zeigte, daß der neue Exon 1 B-Bereich mitten im ersten Intron des Androgenrezeptorgens liegt. Eine Analyse des davor liegenden Genabschnitts zeigt, daß möglicherweise in dieser Region ein zweiter Promotor des Androgenrezeptorgens liegt. Dieser Abschnitt enthält putative Initiatorregionen, die zur Erkennung durch die basale Transkriptionsmaschinerie dienen, sowie mehrere putative Steroidhormon-responsive Elemente.

### Beispiel 2: Gewebeverteilung von AR42 und AR32

Die Gewebeverteilung wurde durch semi-quantitative PCR bestimmt. Die Primer, die für die Isolierung der kompletten AR42 und AR32-cDNA-Sequenzen benutzt wurden (Beispiel 1), wurden auch hier verwendet. In der Kontrolle wurden spezifische Primer für Beta-Actin verwendet (Sense Primer: 5'-TGACGGGGTCACCCACACTGTGCCCATCTA-3'; Antisense Primer: 5'-CTAGAAGCATTTGCGGTGGACGATGGAGGG-3'). Gesamt-RNA aus den folgenden humanen Geweben wurde benutzt: Gehirn, Hoden, Niere, Leber, Uterus, Prostata, Lunge, Trachea, Muskel, Brust, Herz. Nach Umschreiben in First-Strand cDNA (Stratagene) wurde eine PCR-Analyse mit dem Adavantage-2 PCR kit (Clontech) durchgeführt. Die Reaktionsbedingungen waren: 5 Sek. 94 °C, 3 Min. 72 °C (5 Zyklen); 5 Sek. 94 °C, 10 Sek. 70 °C, 3 Min. 72 °C (5 Zyklen); 5 Sek. 94 °C, 10 Sek. 68 °C, 3 Min. 72 °C (20 Zyklen). Die Amplifikationsprodukte wurden auf einem 1%-Agarosegel getrennt und mit Ethidiumbromid gefärbt. Die Ergebnisse zeigten, daß AR42 RNA am häufigsten im Herz, Muskel, Uterus und in der Prostata exprimiert war. Die AR32 RNA-Mengen waren allgemein gering und zeigten keine wesentlichen Unterschiede zwischen Geweben.

### Beispiel 3: Expression von AR42 und AR32

Für die Expression des gesamten AR42 bzw. AR32 wurde der kodierende Bereich in den Baculovirusexpressionsvektor pBlueBac4.5 (Invitrogen) eingefügt. Um Nachweis und Reinigung zu vereinfachen, erfolgte eine Fusion mit einem His-Tag. Nach Cotransfektion von Insektenzellen mit der Bac-N-Blue DNA wurden rekombinante Viren erzeugt, die durch eine PCR-Verfahren identifiziert wurden. Ein Phagenstock wurde dann angelegt und für weitere Transfektionen und Produktion des AR42 bzw. AR32 in größeren Mengen verwendet. Die Reinigung der His-getaggten Proteinen erfolgte über eine Nickel-Affinitätssäule .

### Beispiel 4: Testsystem zum Auffinden von Effektoren

Es wird ein Vektor für die transiente Expression von AR42 bzw. AR32 im pSG5-Plasmid (Stratagene) gebaut. Dieser Vektor wird in CV-1- oder PC-3-Zellen transfiziert. Parallel dazu wird ein Reporterplasmid, das eine oder mehrere Kopien eines Steroid-Response Elements oder eines selektiven Androgen-Response Elements enthält, gekoppelt an ein Luciferase Reportergen, cotransfiziert. Um spezifische Effektoren von AR42 bzw. AR32 zu finden, wird ein Hochdurchsatz-Screening von Substanzbanken durchgeführt. Substanzen die in einer Konzentration von 10⁻⁶ M oder weniger die Aktivität des Reportergens anschalten werden weiter bearbeitet. Das Suchen nach Rezeptorantagonisten wird in Gegenwart von 10⁻⁹ M Androgen, z.B. R1881 durchgeführt. Substanzen die in einer Konzentration von 10⁻⁶ M oder weniger die Androgeninduktion mindestens halbieren werden ausgewählt.

### Beispiel 5: Transreprimierende Aktivität von AR42 und AR32

Es wird ein Vektor für die transiente Expression von AR42, AR32 bzw. AR im pSG5-Plasmid (Stratagene) gebaut. Eine konstante Menge an pSG5-AR und unterschiedliche Mengen an pSG5-AR42 bzw. pSG5-AR32 werden in CV-1-Zellen transfiziert. In der Kontrolle wird ein pSG5-Plasmid, das eine irrelevante cDNA von ähnlicher Länge enthält, mit dem pSG5-AR cotransfiziert. Zusätzlich wird ein Reporterplasmid, das eine oder mehrere Kopien eines Steroid-Response Elements oder eines selektiven Androgen-Response Elements enthält, gekoppelt an ein Luciferase Reportergen, cotransfiziert. Nach Behandlung mit einem Androgen wird eine Erhöhung der Reportergenaktivität gemessen. Steigende Mengen an transfiziertem pSG5-AR42 oder pSG5-AR32 hemmen diese transaktivierenden Effekte von stimuliertem AR.

### Beispiel 6: Nachweis der Expression von AR42 Protein

AR42 oder AR wurden mit dem TNT T7 Quick Coupled Transcription/transtation system (Promega) *in vitro* translatiert. Dafür wurde pSG5-AR42 oder pSG5-AR mit Rabbit reticulocyte lysate Mix bei 30 °C für 90 Minuten inkubiert. Teil des Einsatzes (1/25) oder bzw. der gesamte Zellextrakt (40 ng) wurden auf einem 8% Tris-Glycine Gel getrennt und in Towbin Puffer auf Nitrocellulose Membrane transferiert. Die Transfermembrane wurde in PBS-Tween Puffer mit 5 % Milch blockiert. Der Primärantikörper war ein polyclonaler Antikörper aus Kaninchen, der gegen die Ligand-bindende Domäne des AR gerichtet war. Dieser Antikörper wurde 1/500 in PBS-Tween Puffer mit 3 % Milch verdünnt. Zur Detektion wurde der Amersham ECL Kit verwendet.

### Beispiel 7: RNA Expression in Prostata Tumoren

Die Expression von AR42 und AR32 mRNA in Prostata Tumor wurde durch semi-quantitative PCR bestimmt. Es wurden die in Beispiel 2 beschriebenen Primer verwendet. In der Kontrolle wurden spezifische Primer für humanes ribosomales Protein S9 (Sense Primer: 5'-GATGAGAAGGACCCACGGCGTCTGTTCG-3'; Antisense Primer: 5'-GAGACAATCCAGCAGCCCAGGAGGGACA-3') und für AR (Sense Primer: 5'-CCCTGGATGGATAGCTACTCCGGACCTTACGGGGACATGCGT-3'; Antisense Primer: 5'-TCACTGGGTGTGGAAATAGATGGGCTTGA -3') benutzt. Gesamte RNA aus normalem Prostatagewebe oder aus Prostata Tumor wurde wie im Beispiel 2 analysiert. Die optische Dichte der Banden wurde mit dem Gel Doc System und der Quantity One Software von Biorad gemessen.

## Patentansprüche

1. Nukleinsäure, die für einen Androgenrezeptor kodiert, **dadurch gekennzeichnet, daß** sie die in Seq ID NO 1 und/oder 3 dargestellten Nukleotidsequenzen umfaßt.

2. Nukleinsäure **dadurch gekennzeichnet, daß** sie für ein Polypeptid mit der in Seq ID NO 2 oder/und 4 dargestellten Aminosäuresequenz kodiert.

3. Androgenrezeptor, **dadurch gekennzeichnet, daß** er von einer Nukleinsäure nach einem der Ansprüche 1-2 kodiert ist.

4. Androgenrezeptor, **dadurch gekennzeichnet, daß** er die in Seq ID NO 2 oder 4 dargestellte Aminosäuresequenz umfaßt.

5. Peptid, **dadurch gekennzeichnet, daß** es die in Seq. ID NO 5 dargestellte Sequenz umfaßt.

6. Peptid, **dadurch gekennzeichnet, daß** es die in Seq ID NO 6 dargestellte Aminosäuresequenz umfaßt.

7. Verwendung eines Polypeptids nach Anspruch 3 oder 4 oder eines Peptids nach Anspruch 5 oder/und 6 zur Herstellung von Antikörpern.

8. Antikörper gegen ein Peptid mit der Seq ID NO 5 oder 6.

9. Verwendung eines Antikörpers nach Anspruch 8 zur Detektion eines Polypeptids nach Anspruch 3 oder 4 in Tumorgewebe.

10. Verwendung einer Sonde mit Nukleinsäuresequenzen, die komplementär zu den Nukleinsäuresequenzen sind, die für die Peptide mit einer Aminosäuresequenz gemäß Seq ID 5 oder Seq ID 6 kodieren, zur Herstellung eines Reagenz zum Nachweis der Gegenwart von mRNA nach einem der Ansprüche 1 bis 2 in Tumorzellen.

11. Vektor, **dadurch gekennzeichnet, daß** er mindestens eine Kopie einer Nukleinsäure nach einem der Ansprüche 1-2 enthält.

12. Zelle, **dadurch gekennzeichnet, daß** sie mit einer Nukleinsäure nach einem der Ansprüche 1-2 oder mit einem Vektor nach Anspruch 11 transfiziert ist.

13. Zelle nach Anspruch 12, **dadurch gekennzeichnet, daß** sie aus der Gruppe bestehend aus PC-3-Zellen, LNCaP-Zellen, CV-1-Zellen und Dunning-Zellen ausgewählt ist.

14. Verwendung einer Zelle nach Anspruch 12 oder 13 zur Expression der Nukleinsäure nach einem der Ansprüche 1-2.

15. Verwendung
a. einer Nukleinsäure nach einem der Ansprüche 1 bis2,
b. eines Polypeptids nach Anspruch 3 oder 4,
c. eines Peptids mit der in Seq ID NO 5 dargestellten Aminosäuresequenz oder
d. einer Zelle nach Anspruch 12 oder 13
zur Identifizierung von Inhibitoren oder Aktivatoren der Androgenrezeptorfunktion eines Polypeptids nach Anspruch 3 oder 4.

16. Testsystem zur Detektion von Inhibitoren oder Aktivatoren der Androgenrezeptorfunktion der erfindungsgemäßen Polypeptide, wobei
a. in einer Zelle nach Anspruch 12 oder 13 ein Reportergen exprimiert wird und
b. diese Zelle, wenn sie keinen oder nur wenig eines Polypeptids nach Anspruch 3 oder 4 enthält, zusätzlich mit einem Vektor nach Anspruch 11 transfiziert wird,
c. die Zelle in Gegenwart oder Abwesenheit der Testsubstanzen kultiviert werden und
d. die Veränderung der Expression des Reportergens gemessen wird.

17. Testsystem zur Detektion von Testsubstanzen mit antiandrogener Aktivität, wobei
a. in einer Zelle nach Anspruch 12 oder 13 ein Reportergen exprimiert wird und
b. diese Zelle, wenn sie keinen oder nur wenig eines Polypeptids nach Anspruch 3 oder 4 enthält, zusätzlich mit einem Vektor nach Anspruch 11 transfiziert wird,
c. die Zelle in Gegenwart oder Abwesenheit der Testsubstanzen bei gleichzeitiger Anwesenheit eines Androgens kultiviert wird und
d. die Veränderung der Expression des Reportergens gemessen wird.

## Claims

1. Nucleic acid that codes for an androgen receptor, **characterized in that** it comprises the nucleotide sequences that are shown in Seq ID NO 1 and/or 3.

2. Nucleic acid, **characterized in that** it codes for a polypeptide with the amino acid sequence that is shown in Seq ID NO 2 and/or 4.

3. Androgen receptor, **characterized in that** it is coded by a nucleic acid according to one of claims 1-2.

4. Androgen receptor, **characterized in that** it comprises the amino acid sequence that is shown in Seq ID NO 2 or 4.

5. Peptide, **characterized in that** it comprises the sequence that is shown in Seq. ID NO 5.

6. Peptide, **characterized in that** it comprises the amino acid sequence that is shown in Seq. ID NO 6.

7. Use of a polypeptide according to claim 3 or 4 or a peptide according to claim 5 and/or 6 for the production of antibodies.

8. Antibody against a peptide having SEQ ID No 5 or 6.

9. Use of an antibody according to claim 8 for detection of a polypeptide according to claim 3 or 4 in the tumor tissue.

10. Use of a probe with nucleic acid sequences that are complementary to the nucleic acid sequences, that code for the peptides according to Seq ID 5 or Seq ID 6, for the production of a reagent for detecting the presence of mRNA according to one of claims 1 to 2 in tumor cells.

11. Vector, **characterized in that** it contains at least one copy of a nucleic acid according to one of claims 1-2.

12. Cell, **characterized in that** it is transfected with a nucleic acid according to one of claims 1-2 or with a vector according to claim 11.

13. Cell according to claim 12, **characterized in that** it is selected from the group that consists of PC-3 cells, LNCaP cells, CV-1 cells, CV-1 cells and Dunning cells.

14. Use of a cell according to claim 12 or 13 for the expression of nucleic acid according to one of claims 1-2.

15. Use of
a. a nucleic acid according to one of claims 1 to 2,
b. a polypeptide according to claim 3 or 4,
c. a peptide with the amino acid sequence that is shown in Seq ID NO 5 or
d. a cell according to claim 12 or 13 to identify inhibitors or activators of the androgen receptor function of a polypeptide according to claim 3 or 4.

16. Test system for detecting inhibitors or activators of the androgen receptor function of the polypeptides according to the invention, whereby
a. a reporter gene is expressed in a cell according to claim 12 or 13, and
b. this cell, if it contains only a little or no polypeptide according to claim 3 or 4, is transfected in addition with a vector according to claim 11,
c. the cell is cultivated in the presence or absence of the test substances, and
d. the alteration of the expression of the reporter gene is measured.

17. Test system for detecting test substances with antiandrogenic activity, whereby
a. a reporter gene is expressed in a cell according to claim 12 or 13, and
b. this cell, if it contains only a little or no polypeptide according to claim 3 or 4, is transfixed in addition with a vector according to claim 11,
c. the cell is cultivated in the presence or absence of the test substances with the simultaneous presence of an androgen, and
d. the alteration of the expression of the reporter gene is measured.

## Revendications

1. Acide nucléique, qui code pour un récepteur d'androgène, **caractérisé en ce qu'**il comporte les séquences de nucléotides représentées par SEQ ID n° 1 et/ou 3.

2. Acide nucléique, **caractérisé en ce qu'**il code pour un polypeptide comportant la séquence d'acides aminés représentée par SEQ ID n° 2 et/ou 4.

3. Récepteur d'androgène, **caractérisé en ce qu'**il est codé par un acide nucléique selon l'une des revendications 1 à 2.

4. Récepteur d'androgène, **caractérisé en ce qu'**il comporte la séquence d'acides aminés représentée par SEQ ID n° 2 ou 4.

5. Peptide, **caractérisé en ce qu'**il comporte la séquence représentée par SEQ ID n° 5.

6. Peptide, **caractérisé en ce qu'**il comporte la séquence d'acides aminés représentée par SEQ ID n° 6.

7. Utilisation d'un polypeptide selon la revendication 3 ou 4 ou d'un peptide selon la revendication 5 et/ou 6 pour la fabrication d'anticorps.

8. Anticorps contre un peptide comportant la SEQ ID n° 5 ou 6.

9. Utilisation d'un anticorps selon la revendication 8 pour la détection d'un polypeptide selon la revendication 3 ou 4 dans des tissus tumoraux.

10. Utilisation d'une sonde avec des séquences d'acides nucléiques qui sont complémentaires des séquences d'acides nucléiques qui codent pour les peptides comportant une séquence d'acides aminés selon SEQ ID n° 5 ou SEQ ID n° 6, pour la fabrication d'un réactif pour la détection de la présence d'ARNm selon l'une quelconque des revendications 1 à 2 dans des cellules tumorales.

11. Vecteur, **caractérisé en ce qu'**il contient au moins une copie d'un acide nucléique selon l'une des revendications 1 à 2.

12. Cellule, **caractérisée en ce qu'**elle est transfectée avec un acide nucléique selon une des revendications 1 à 2 ou avec un vecteur selon la revendication 11.

13. Cellule selon la revendication 12, **caractérisée en ce qu'**elle est sélectionnée parmi le groupe constitué de cellules PC-3, de cellules LNCaP, de cellules CV-1, de cellules CV-1 et de cellules Dunning.

14. Utilisation d'une cellule selon la revendication 12 ou 13 pour l'expression de l'acide nucléique selon l'une des revendications 1 à 2.

15. Utilisation
a. d'un acide nucléique selon l'une des revendications 1 à 2,
b. d'un polypeptide selon la revendication 3 ou 4,
c. d'un peptide comportant la séquence d'acides aminés représentée par SEQ ID n° 5 ou
d. d'une cellule selon la revendication 12 ou 13
pour l'identification d'inhibiteurs ou d'activateurs de la fonction réceptrice d'androgène d'un polypeptide selon la revendication 3 ou 4.

16. Système de test pour la détection d'inhibiteurs ou d'activateurs de la fonction récepteur d'androgène des polypeptides selon la présente invention, dans lequel :
a. un gène rapporteur est exprimé dans une cellule selon la revendication 12 ou 13 et
b. cette cellule, si elle ne contient pas ou si elle contient seulement peu d'un polypeptide selon la revendication 3 ou 4, est transfectée de manière supplémentaire avec un vecteur selon la revendication 11,
c. la cellule est cultivée en présence ou en l'absence des substances de test et
d. la modification de l'expression du gène rapporteur est mesurée.

17. Système de test pour la détection de substances de test ayant une activité antiandrogène, dans lequel :
a. un gène rapporteur est exprimé dans une cellule selon la revendication 12 ou 13 et
b. cette cellule, si elle ne contient pas ou si elle contient seulement peu d'un polypeptide selon la revendication 3 ou 4, est transfectée de manière supplémentaire avec un vecteur selon la revendication 11,
c. la cellule est cultivée en présence ou en l'absence des substances de test avec présence simultanée d'un androgène et
d. la modification de l'expression du gène rapporteur est mesurée.
